# EUROPÄISCHE PATENTSCHRIFT

(11) **EP 0 300 311 B1**
(45) Veröffentlichungstag und Bekanntmachung des Hinweises auf die Patenterteilung: **21.09.1994**
(21) Anmeldenummer: 88111034.0
(22) Anmeldetag: 11.07.1988
(51) Int. Cl.: C07D 215/56

(54) **Verfahren zur Herstellung von Chinoloncarbonsäuren**
Method for the preparation of quinolone carboxylic acids
Procédé de préparation d'acides quinoloncarboxyliques

(30) Priorität: 24.07.1987 DE 3724466
(43) Veröffentlichungstag der Anmeldung: 25.01.1989
(73) Patentinhaber: BAYER AG, 51368 Leverkusen (DE)
(72) Erfinder: Zerbes, Rudolf, Dr., D-5600 Wuppertal 1 (DE); Preiss, Michael, Dr., D-5600 Wuppertal 1 (DE)

(56) Entgegenhaltungen:
- EP-A- 0 004 279
- EP-A- 0 078 362
- EP-A- 0 168 733
- EP-A- 0 176 846
- EP-A- 0 216 245

## Beschreibung

Die Erfindung betrifft ein neues Verfahren zur Herstellung von 4-Oxo-3-chinolin-carbonsäuren, die als Zwischenprodukte für die Herstellung bekannter pharmazeutisch wirksamer Chinoloncarbonsäuren Verwendung finden.

Es ist bekannt, daß man 1-Cyclopropyl-7-chlor-6-fluor-1,4-dihydro-4-oxo-3-chinolin-carbonsäuren auf folgendem Wege erhält (EP-A-0 176 846): 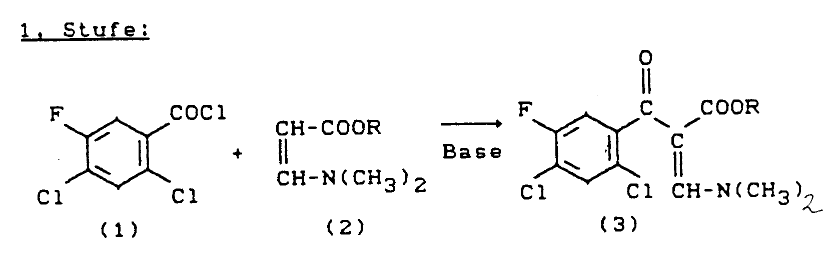 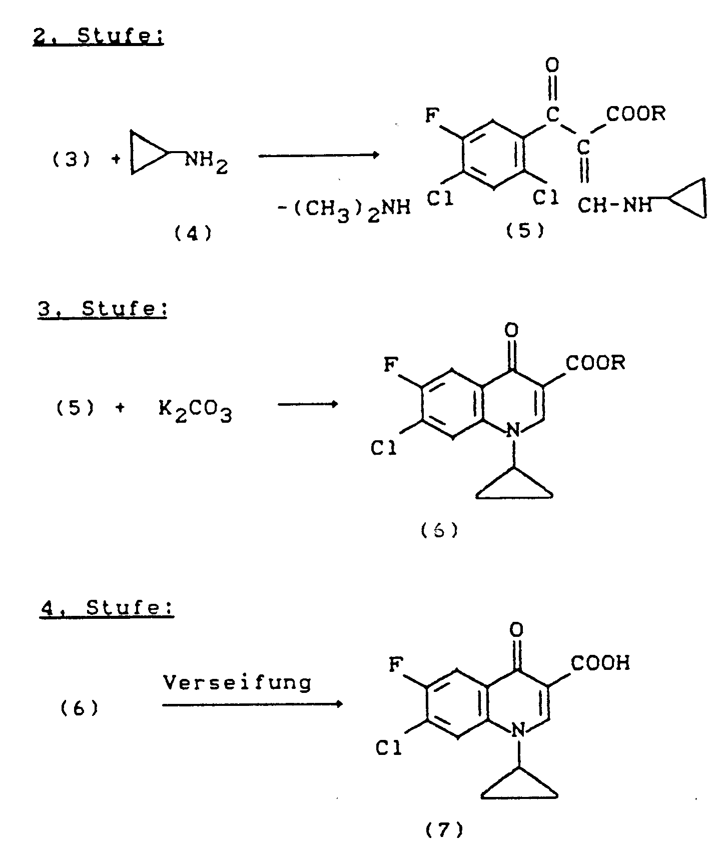
Es ist auch bekannt geworden, die Verbindung (7) auf folgendem Wege herzustellen: (EP-A-0 078 362) 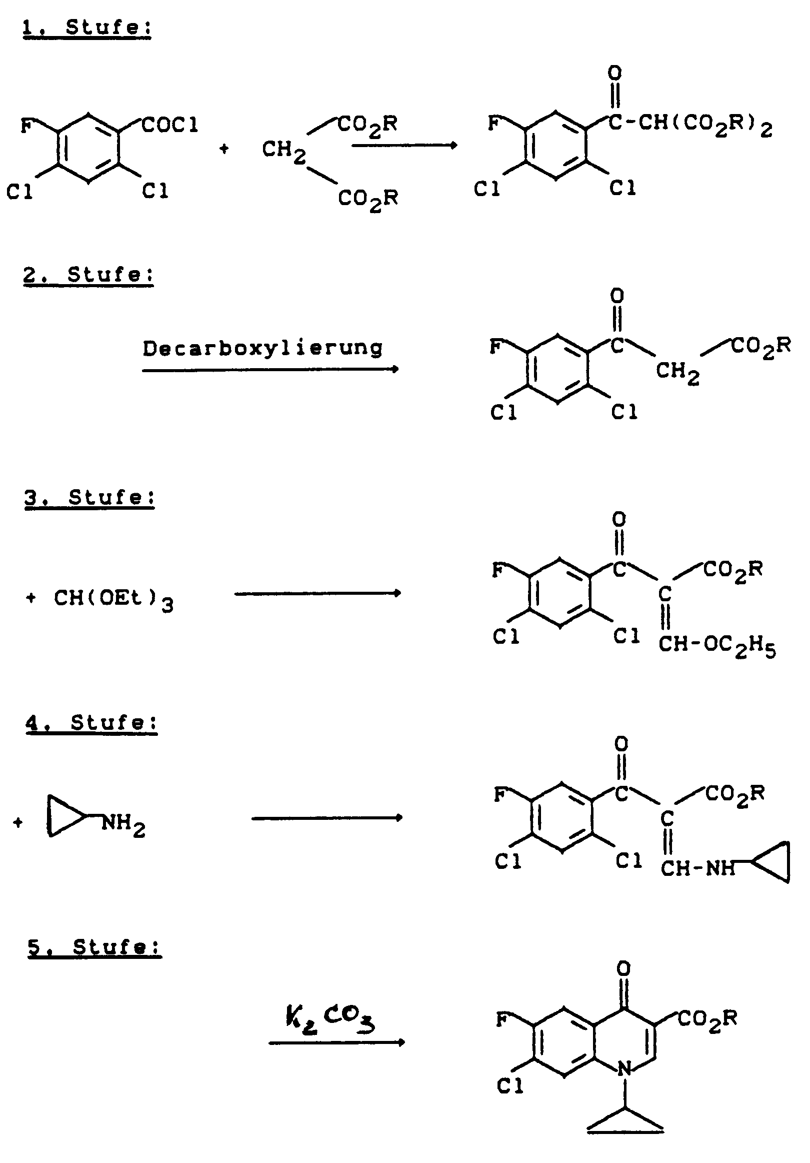 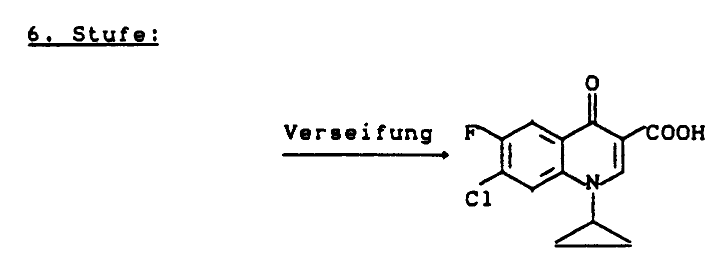
Die bekannten Verfahren weisen jedoch alle den Nachteil auf, über viele Zwischenstufen die Zielverbindung herzustellen.

Dabei müssen aufwendige Trenn- und Trockenoperationen durchgeführt werden.

Durch die dabei notwendigen Waschvorgänge fallen große Mengen an Lösungsmitteln an, die entweder verbrannt oder wieder aufbereitet werden müssen.

Ferner muß eine aufwendige Analytik zur Charakterisierung der Zwischenstufen getrieben werden.

Die Erfindung betrifft ein Verfahren zur Herstellung von Chinoloncarbonsäuren der allgemeinen Formel I 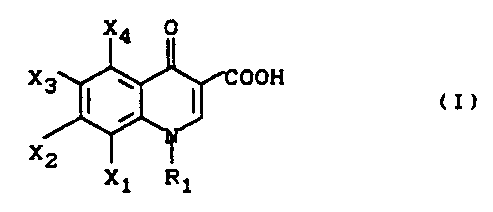
in der
- R¹: für Propyl, Cyclopropyl, Isopropyl oder Vinyl steht,
- X¹: Fluor, Chlor, Br, CN, NO₂ oder Wasserstoff bedeutet und
X², X³ und X⁴ für Fluor, Chlor, NO₂ oder Wasserstoff stehen,
das dadurch gekennzeichnet ist, daß man ohne Isolierung der Reaktionsprodukte der Zwischenstufen in einer sogenannten Eintopfreaktion eine Verbindung der allgemeinen Formel II 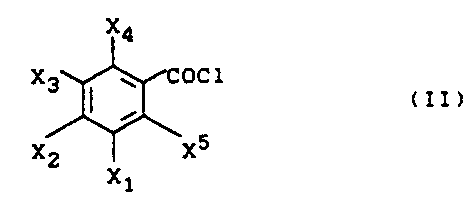
in der X¹ bis X⁴ die obengenannten Bedeutungen haben und X⁵ für Halogen, insbesondere Chlor oder Fluor steht, mit einer Verbindung der allgemeinen Formel III 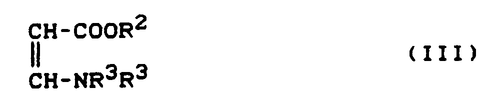
in der
- R² und R³: gleich oder verschieden sind und für C₁-C₄-Alkyl stehen,
in Gegenwart eines Lösungsmittels und einer Base, gegebenenfalls unter Erwärmung auf 50°C bis 150°C zu einer Verbindung der allgemeinen Formel IV 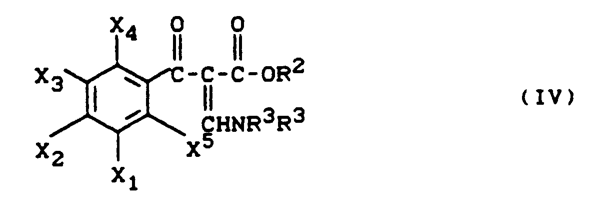
umsetzt,
diese Verbindung IV bei Temperaturen von 50 bis 120°C in Gegenwart der obengenannten Lösungsmittel und in Gegenwart eines Amins der allgemeinen Formel R₁NH₂, worin R¹ die obengenannte Bedeutung hat, einem Aminaustausch unterwirft, wobei eine Verbindung der allgemeinen Formel V 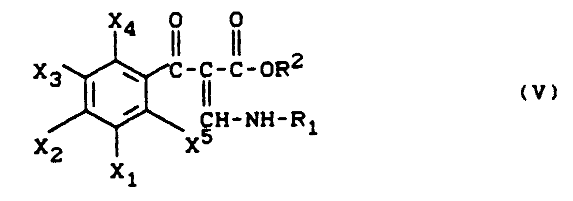
mit den obengenannten Restebedeutungen entsteht, und anschließend die Verbindung V bei Temperaturen zwischen 80 und 180°C in Gegenwart einer Base cyclisiert, verseift und durch Zugabe von Säure eine Verbindung der allgemeinen Formel I ausfällt.

Insbesondere werden erfindungsgemäß solche Verbindungen hergestellt, in denen R¹ in der allgemeinen Formel I für einen Cyclopropylrest steht.
- Bevorzugt:: X¹ und X⁴ = Wasserstoff
X² = Chlor
X³ = Fluor.

Bevorzugt sind auch Verbindungen der allgemeinen Formel I, in denen R¹ Cyclopropyl bedeutet, X² für Chlor steht und X¹ und X³ Fluor bedeuten, während X⁴ für Wasserstoff steht.

Weiterhin bevorzugt sind Verbindungen, in denen X³ für Fluor und X¹ und X² für Chlor stehen, während X⁴ Wasserstoff bedeutet.

Bei dem komplizierten Reaktionsverlauf ist es als ausgesprochen überraschend zu bezeichnen, daß man z.B. die Verbindung 1-Cyclopropyl-7-chlor-6-fluor-1,4-dihydro-4-oxo-3-chinolin-carbonsäure ohne Isolierung von Zwischenstufen produkten in einer "Eintopf"-Reaktion in ausgezeichneten Ausbeuten auf folgendem Wege herstellen kann:

### a) Acylierung

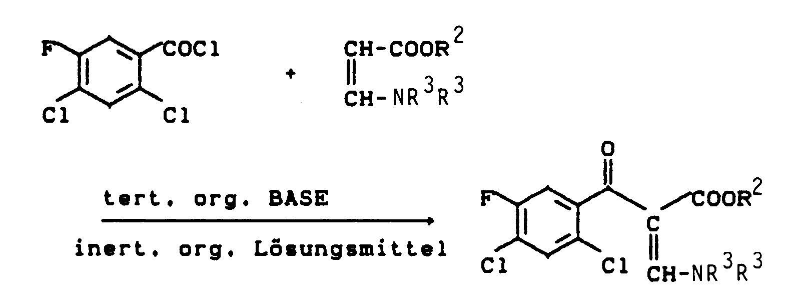

Als org. Lösungsmittel kommen in Frage:
Toluol,
Xylol,
Cyclohexan,
offenkettige Kohlenwasserstoffe (Gemische),
DMF,
DMSO.

Als Basen können eingesetzt werden:
tert. org. Amine wie
R₃N mit R = C₁-C₄-Alkyl, Benzyl,
cycl. Amine 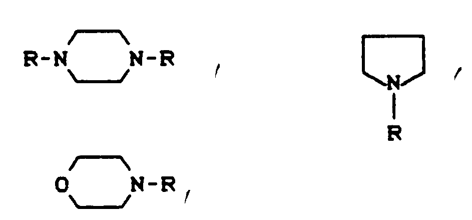
Pyridin etc. mit R = C₁-C₄-Alkyl.

Die Temperaturen bei der Acylierung des Dimethylaminoacrylsäureesters liegen zwischen 50 und 150°C, vorzugsweise bei 80 bis 120°C.

Während der Reaktion ausgefallenes Hydrochlorid der Hilfsbase kann über ein Filter abgetrennt oder durch Ausrühren mit Wasser entfernt werden.

Die org. Phase mit dem Aroylacrylsäureester wird weiter mit Cyclopropylamin umgesetzt.

Dabei kann die Umsetzung im gleichen Lösungsmittel, oder nach Eindampfen (i. Vakuum oder bei Normaldruck) in einem anderen Lösungsmittel erfolgen.

### b) Aminaustausch:

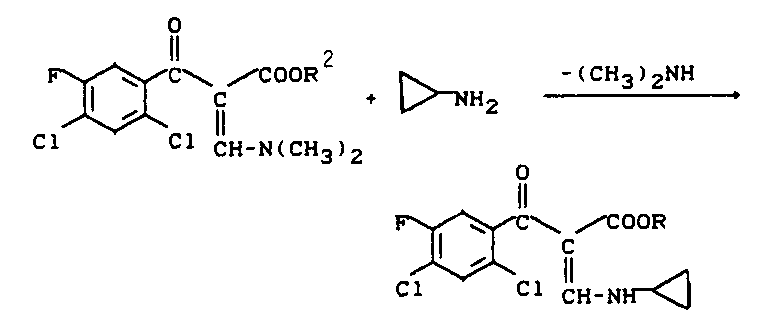

Als Lösungsmittel kommen in Frage:
Toluol,
Xylol,
Cyclohexan,
offenkettige Kohlenwasserstoffe (Gemische),
Alkohole,
DMF,
DMSO,
Butylglykol.

Die Temperaturen beim Aminaustausch liegen bei 50 bis 120°C, vorzugsweise bei 65 bis 85°C.

Das Reaktionsgemisch wird weiter bei der erhöhten Temperatur gehalten, bis die Gasentwicklung (Dimethylamin) beendet ist.

Falls der Aminaustausch in einem niedrigsiedenden Verdünnungsmittel, wie z.B. Cyclohexan erfolgt, wird das Lösungsmittel vor der Cyclisierung (i. Vakuum, oder bei Normaldruck) abgedampft und durch ein höhersiedendes Verdünnungsmittel, wie z.B. Butylglykol ersetzt.

### c) Cyclisierung:

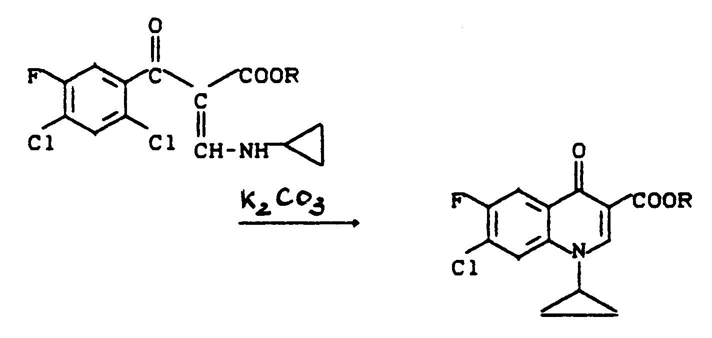

Als Lösungsmittel kommen in Frage:
höhere Alkohole, wie
Butylglykol, i-Propylalkohol,
Ethylenglykol, Butanol,
Triethylenglykol,
Polyethylenglykol,
Aminoalkohole wie Diethylaminoethanol,
DMF,
DMSO,
Dioxan,
N-Methylpyrrolidon.

Die Cyclisierung zum Chinolonsystem erfolgt bei Temperaturen zwischen 80 und 180°C, vorzugsweise bei 130 bis 160°C in Gegenwart einer Base.

Als Basen können eingesetzt werden:
Na-tert.-butylat,
NaH,
K₂CO₃.

Die Base wird äquimolar bis zu einen Überschuß von 3 Moläquivalenten, vorzugsweise in einem Überschuß von 0,1 bis 0,5 Moläquivalenten eingesetzt.

### d) Verseifung und Fällung

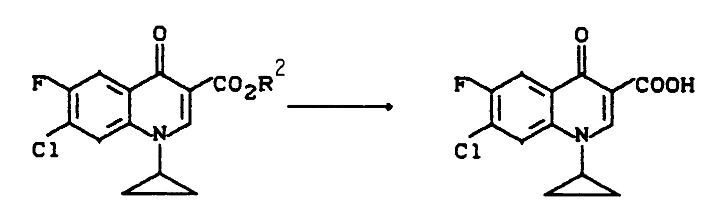

Zur Verseifung wird die Reaktionsmischung auf ca. 100°C abgekühlt und mit Wasser versetzt.

Durch den Überschuß an Base bei der Cyclisierung ist die Verseifung bei Temperaturen von 50 bis 100°C in kurzer Zeit beendet.

Die Chinoloncarbonsäure wird durch Zugabe einer Mineralsäure oder einer org. Säure ausgefällt und isoliert.

Als Säuren kommen bevorzugt in Frage:
Schwefelsäure,
Salzsäure,
Essigsäure.

### Beispiel 1

28,8 g N,N-Dimethylamino-acrylsäureethylester und 26 g N,N-Dimethylbenzylamin werden in 71 ml Toluol auf 90°C erhitzt und bei dieser Temperatur 40 g 2,4-Dichlor-5-fluorbenzoylchlorid in 60 Min. zugetropft.

Anschließend wird 15 Min. nachgerührt und das ausgefallene N,N-Dimethylbenzylamin-hydrochlorid über eine Filternutsche abgetrennt.

Das Filtrat wird im Vakuum weitgehend eingedampft und der Rückstand mit 80 ml Butylglykol versetzt.

Bei 70-75°C läßt man in 30 Min. 13 g Cyclopropylamin zutropfen und hält nach dem Ende der Zugabe den Ansatz 1 Stunde lang bei 100°C bis zum Ende der Gasentwicklung.

Zum Reaktionsgemisch gibt man 27,9 g Pottasche und 100 ml Butylglykol und erhitzt langsam auf 135-145°C.

Dabei destillieren ca. 40 ml leichtsiedende Anteile ab.

Die Temperatur wird 1,5 Std. lang gehalten.

Anschließend kühlt man auf 100-120°C ab und gibt 130 ml Wasser zum Reaktionsgemisch.

Man rührt 15 Min. bei 90°C nach und tropft bei dieser Temperatur 27 ml Essigsäure in 15 Min. zu. Der Kolbeninhalt wird abgekühlt und der Feststoff über eine Filternutsche abgetrennt.

Das Produkt wird mit 27 ml Wasser und 50 ml Methanol gewaschen, gut trockengesaugt und 24 Std. lang bei 70°C i.Vak. getrocknet.
Ausbeute: 37 g = 74,9 % d.Th.

### Beispiel 2

43,2 g N,N-Dimethylamino-acrylsäureethylester werden in 84 ml Toluol auf 100°C aufgeheizt und 40 ml Triethylamin zugegeben.

Bei Rückflußtemperatur läßt man in 30 Min. 60 g 2,4-Dichlor-5-fluorbenzoylchlorid zutropfen und trennt anschließend das ausgefallene Triethylamin-hydrochlorid über eine Filternutsche ab.

Man wäscht mit 65 ml Toluol nach und tropft zum Filtrat bei 70°C 16,2 g Cyclopropylamin in 20 Min. zu.

Man erhitzt auf 100°C bis zum Ende der Gasentwicklung.

Anschließend werden 42 g Pottasche und 270 ml Butylglykol zugegeben und langsam auf 135-145°C aufgeheizt. Dabei destillieren ca. 180 ml Toluol und niedrigsiedende Anteile ab.

Die Temperatur wird 1,5 Std. lang auf 135 - 145°C. gehalten und nach Abkühlen der Reaktionsmischung auf 100°C werden 200 ml Wasser zugegeben.

Nach 15 Min. werden bei 90°C 40,5 ml Essigsäure zugetropft und der ausgefallene Feststoff über eine Filternutsche abgesaugt.

Das Produkt wird mit 140 ml Wasser und 75 ml Methanol nachgewaschen, gut trockengesaugt und 24 Std. lang bei 70°C i. Vakuum getrocknet.
Ausbeute: 58,5 g = 79 % d.Th.

### Beispiel 3

44,7 g N,N-Dimethylamino-acrylsäureethylester und 17,1 g N,N′-Dimethylpiperazin werden in 95 ml Cyclohexan zum Rückfluß erhitzt.

In 1 Std. werden 62 g 2,4-Dichlor-5-fluorbenzoylchlorid zugetropft.

Das ausgefallene Dimethylbenzylamin-hydrochlorid wird über eine Filternutsche abgetrennt und das Filtrat i. Vakuum zur Trockne eingedampft.

Der Rückstand wird in 125 ml Butylglykol gelöst und zu der Lösung werden bei 90°C 20,2 g Cyclopropylamin zugetropft.

Nach dem Ende der Gasentwicklung gibt man 43,4 g Pottasche und 165 ml Butylglykol zu und erwärmt 1,5 Std. lang auf 145°C. Dabei destillieren anfangs geringe Mengen leichtsiedende Anteile ab.

Nach 1,5 Std. wird auf 100°C abgekühlt, mit 205 ml Wasser versetzt und 15 Min. lang bei 95°C nachgerührt.

Anschließend läßt man 42 g Essigsäure in 15 Min. zutropfen, kühlt ab auf 30°C und trennt den ausgefallenen Feststoff über eine Filternutsche ab.

Der Filterkuchen wird mit 180 ml Wasser und 80 ml 80 %igem Isopropanol gewaschen und bei 70°C über Nacht i.Vak. getrocknet.
Ausbeute: 60,1 g = 78,1 % d.Th.

### Beispiel 4

33,7 g N,N-Dimethylamino-acrylsäuremethylester werden mit 40,5 g N,N-Dimethylbenzylamin in 95 ml Toluol auf 110°C erhitzt.

Bei Rückflußtemperatur werden 62 g 2,4-Dichlor-5-fluorbenzoylchlorid in 1 Std. zugetropft und anschließend das ausgefallene Dimethylbenzylamin-hydrochlorid über eine Filternutsche abgetrennt.

Das Lösungsmittel wird i.Vak. abdestilliert, 130 ml Butylglykol zugegeben und bei 90°C 20,2 g Cyclopropylamin in 20 Min. zugetropft.

Nach dem Ende der Gasentwicklung versetzt man das Reaktionsgemisch mit 43,4 g Pottasche und 150 ml Butylglykol, erhitzt langsam auf 140°C. Dabei destillieren geringe Mengen niedrigsiedender Lösungsmittel ab.

1,5 Std. lang wird bei 140°C nachgerührt. Nach Abkühlen auf 100°C gibt man 205 ml Wasser zum Reaktionsgemisch und läßt 15 Min. lang bei 90°C rühren.

Unter leichter Kühlung werden 100 ml 30 %iger Schwefelsäure zugegeben und der Feststoff über eine Filternutsche abgesaugt, mit 200 ml Wasser und 200 ml Isopropanol gewaschen.

Der Feststoff wird i.Vak. über Nacht bei 70°C getrocknet.
Ausbeute: 59.4 g = 77,2 % d.Th.

### Beispiel 5

Zu 44,7 g N,N-Dimethylamino-acrylsäureethylester in 95 ml Cyclohexan gibt man 55,6 g Tributylamin und tropft bei 85-95°C 62 g 2,4-Dichlor-5-fluorbenzoylchlorid in 1 h zu.

Zum Reaktionsgemisch gibt man anschließend 250 ml Wasser und trennt die wäßrige Salzphase ab.

Die wäßrige Phase wird noch 1 x mit 50 ml Cyclohexan ausgerührt und die vereinigten org. Phasen im Wasserstrahlvakuum zur Trockne eingedampft.

Den Rückstand nimmt man mit 125 ml Butylglykol auf, erwärmt auf 70°C und tropft in 15 Min. 20.2 g Cyclopropylamin zu.

Nach dem Ende der Gasentwicklung werden weitere 160 ml Butylglykol und 44 g Pottasche zugegeben und der Ansatz langsam auf 140°C aufgeheizt.

Nach Erreichen von 140°C wird 1,5 Std. lang nachgerührt, anschließend auf 100°C abgekühlt und 205 ml Wasser zum Ansatz gegeben.

Nach 15 Min. tropft man in 10 Min. 42 g Essigsäure zu und kühlt auf Raumtemperatur ab.

Der Niederschlag wird über eine Filternutsche isoliert und mit Wasser und Isopropanol gewaschen.

Nach Trocknen bei 50°C i. Vak. über Nacht erhält man 54,6 g = 71 % d. Th. Ausbeute.

### Beispiel 6

In 148 ml Toluol werden 105 g Tributylamin und 86 g N,N-Dimethylamino-acrylsäuremethylester auf 105°C erhitzt und in 1 Std. 124 g 2,4-Dichlor-5-fluorbenzoylchlorid zugetropft.

Nach dem Abkühlen des Reaktionsgemisches auf 50 °C wird 2 x mit je 250 ml Wasser extrahiert.

Die organische Phase wird im Wasserstrahlvakuum weitgehend eingeengt und in den Rückstand bei 70 - 75°C Innentemperatur 37,5 g Cyclopropylamin zugetropft. Dabei entweicht bis zum Ende der Reaktion gasförmiges Dimethylamin.

Das Reaktionsgemisch wird mit 86,8 g Pottasche und 500 ml Butylglykol versetzt und langsam bis auf 135 - 145°C erhitzt. Dabei destillieren geringe Mengen niedrigsiedender Lösungsmittel ab.

Die Temperatur wird 1,5 Stdn. lang auf 135 - 145°C gehalten, dann auf 100°C abgekühlt und 535 ml einer 10%-igen Essigsäure werden zugetropft.

Die dadurch entstandene Suspension wird auf Raumtemperatur abgekühlt, der Feststoff über eine Filternutsche abgetrennt und mit 175 ml Wasser und 200 ml 80%-igem Isopropanol gewaschen.

Das Produkt wird im Vakuum über Nacht bei 70°C getrocknet.
Ausbeute: 220 g = 78.1 % d. Th.

## Patentansprüche

1. Verfahren zur Herstellung von Chinoloncarbonsäuren der allgemeinen Formel I 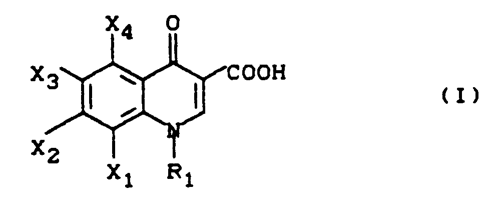 in der
R¹ für Propyl, Cyclopropyl, Isopropyl oder Vinyl steht,
X¹ Fluor, Chlor, Br, CN, NO₂ oder Wasserstoff bedeutet und
X², X³ und X⁴ für Fluor, Chlor, NO₂ oder Wasserstoff stehen,
dadurch gekennzeichnet, daß man ohne Isolierung von Reaktionsprodukten der Zwischenstufen in einer sogenannten Eintopfreaktion eine Verbindung der allgemeinen Formel II 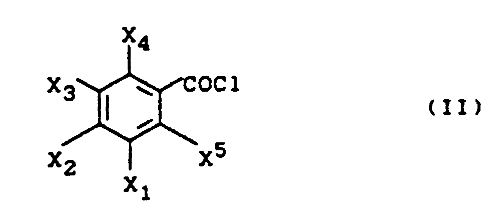 in der X¹ bis X⁴ die obengenannte Bedeutung haben und
X⁵ für Halogen, insbesondere Chlor oder Fluor steht, mit einer Verbindung der allgemeinen Formel III 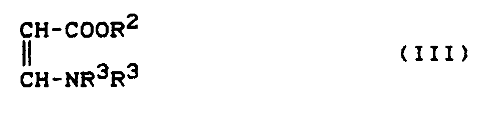 in der
R² und R³ gleich oder verschieden sind und für C₁-C₄-Alkyl stehen,
in Gegenwart eines Lösungsmittels und einer Base, gegebenenfalls unter Erwärmung auf 50 bis 150°C zu einer Verbindung der allgemeinen Formel IV 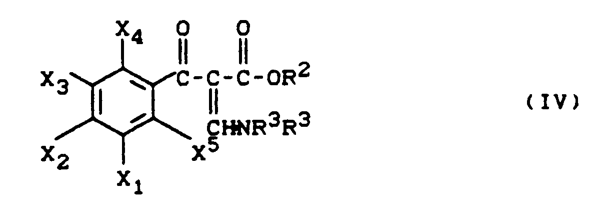 umsetzt,
diese Verbindung IV bei Temperaturen von 50 bis 120°C in Gegenwart der obengenannten Lösungsmittel und in Gegenwart eines Amins der allgemeinen Formel R₁NH₂, worin R¹ die obengenannte Bedeutung hat, einem Aminaustausch unterwirft, wobei eine Verbindung der allgemeinen Formel V 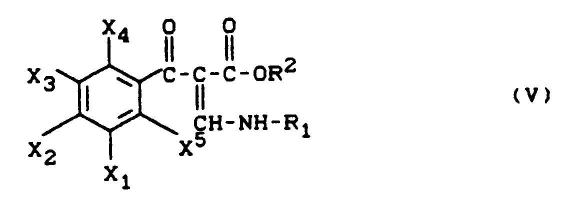 mit den obengenannten Restebedeutungen entsteht, und anschließend die Verbindung V bei Temperaturen zwischen 80 und 180°C in Gegenwart einer Base cyclisiert, verseift und durch Zugabe von Säure die Verbindung der allgemeinen Formel I ausfällt.

2. Verfahren zur Herstellung von Chinoloncarbonsäuren der allgemeinen Formel I 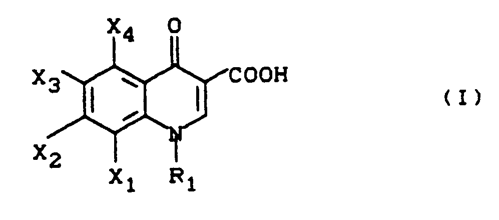 in der
R¹ für Cyclopropyl steht,
X¹ Fluor, Chlor, Br, CN, NO₂ oder Wasserstoff bedeutet und
X², X³ und X⁴ für Fluor, Chlor, NO₂ oder Wasserstoff stehen,
dadurch gekennzeichnet, daß man ohne Isolierung von Reaktionsprodukten der Zwischenstufen in einer sogenanten Eintopfreaktion eine Verbindung der allgemeinen Formel II 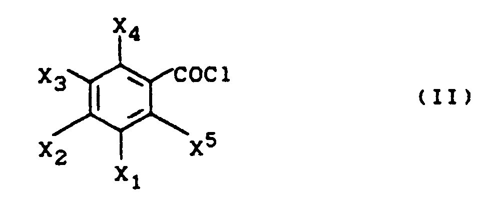 in der X¹ bis X⁴ die obengenannte Bedeutung haben und
X⁵ für Halogen, insbesondere Chlor oder Fluor steht, mit einer Verbindung der allgemeinen Formel III 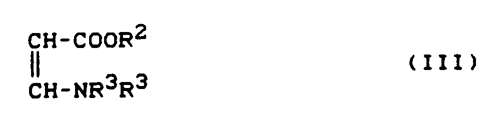 in der
R² und R³ gleich oder verschieden sind und für C₁-C₄-Alkyl stehen,
in Gegenwart eines Lösungsmittels und einer Base, gegebenenfalls unter Erwärmung auf 50 bis 150°C zu einer Verbindung der allgemeinen Formel IV 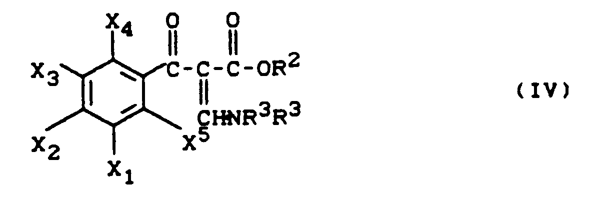 umsetzt,
diese Verbindung IV bei Temperaturen von 50 bis 120°C in Gegenwart der obengenannten Lösungsmittel und in Gegenwart von Cyclopropylamin einem Aminaustausch unterwirft, wobei eine Verbindung der allgemeinen Formel V 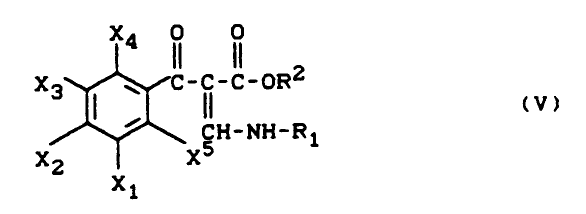 mit den obengenannten Restebedeutungen entsteht, und anschließend die Verbindung V bei Temperaturen zwischen 80 und 180°C in Gegenwart einer Base cyclisiert, verseift und durch Zugabe von Säure die Verbindung der allgemeinen Formel I ausfällt.

3. Verfahren zur Herstellung von Chinoloncarbonsäuren der allgemeinen Formel I 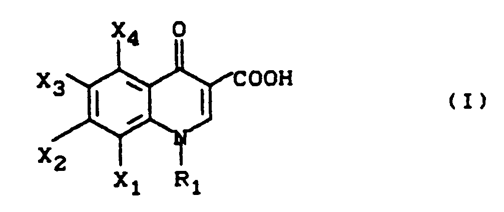 in der
R¹ für Cyclopropyl steht,
X¹, X² und X³ gleich oder verschieden sind und für Fluor oder Chlor stehen und
X⁴ für Wasserstoff steht,
dadurch gekennzeichnet, daß man ohne Isolierung von Reaktionsprodukten der Zwischenstufen in einer sogenannten Eintopfreaktion eine Verbindung der allgemeinen Formel II 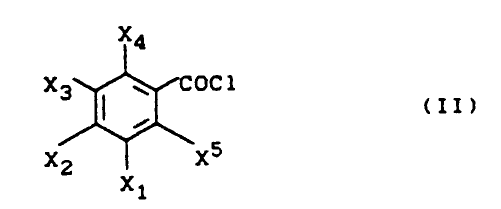 in der X¹ bis X⁴ die obengenannte Bedeutung haben und
X⁵ für Halogen, insbesondere Chlor oder Fluor steht, mit einer Verbindung der allgemeinen Formel III 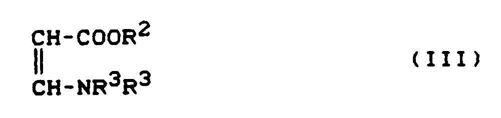 in der
R² und R³ gleich oder verschieden sind und für C₁-C₄-Alkyl stehen,
in Gegenwart eines Lösungsmittels und einer Base, gegebenenfalls unter Erwärmung auf 50 bis 150°C zu einer Verbindung der allgemeinen Formel IV 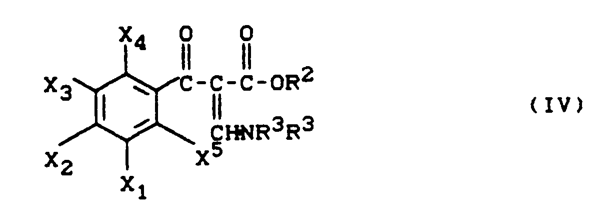 umsetzt,
diese Verbindung IV bei Temperaturen von 50 bis 120°C in Gegenwart der obengenannten Lösungsmittel und in Gegenwart von Cyclopropylamin einem Aminaustausch unterwirft, wobei eine Verbindung der allgemeinen Formel V 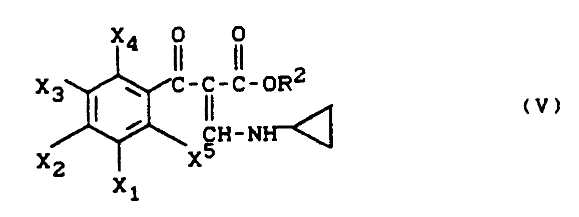 mit den obengenannten Restebedeutung entsteht, und anschließend die Verbindung V bei Temperaturen zwischen 80 und 180°C in Gegenwart einer Base cyclisiert, verseift und durch Zugabe von Säure die Verbindung der allgemeinen Formel I ausfällt.

4. Verfahren zur Herstellung von Chinoloncarbonsäuren der allgemeinen Formel I 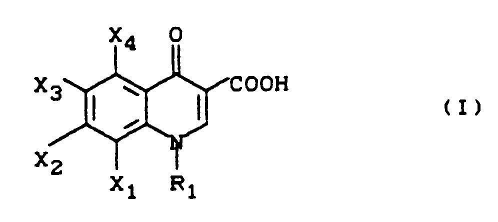 in der
R¹ für Cyclopropyl steht,
X¹ und X⁴ Wasserstoff bedeuten,
X₃ für Fluor steht und
X² Chlor bedeutet,
dadurch gekennzeichnet, daß man ohne Isolierung von Reaktionsprodukten der Zwischenstufen in einer sogenannten Eintopfreaktion eine Verbindung der allgemeinen Formel II 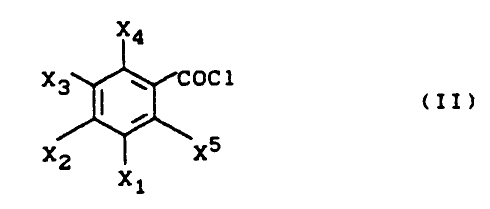 in der X¹ bis X⁴ die obengenannte Bedeutung haben und
X⁵ für Halogen, insbesondere Chlor oder Fluor steht, mit einer Verbindung der allgemeinen Formel (III) 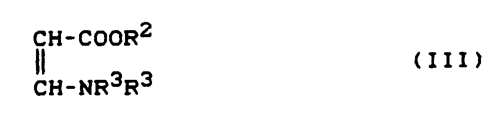 in der
R² und R³ gleich oder verschieden sind und für C₁-C₄-Alkyl stehen,
in Gegenwart eines Lösungsmittels und einer Base, gegebenenfalls unter Erwärmung auf 50 bis 150°C zu einer Verbindung der allgemeinen Formel IV 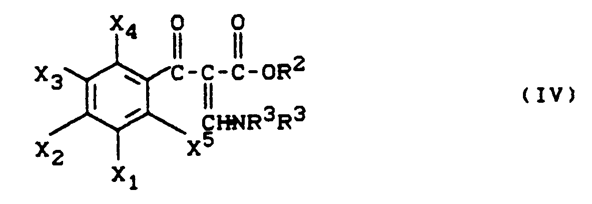 umsetzt,
diese Verbindung IV bei Temperaturen von 50 bis 120°C in Gegenwart der obengenannten Lösungsmittel und in Gegenwart von Cyclopropylamin einem Aminaustausch unterwirft, wobei eine Verbindung der allgemeinen Formel V 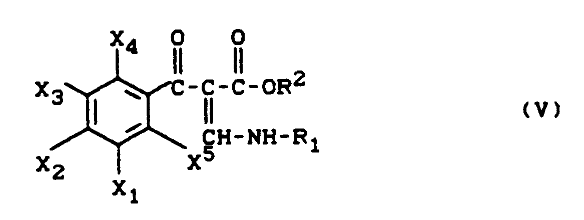 mit den obengenannten Restebedeutungen entsteht, und anschließend die Verbindung V bei Temperaturen zwischen 80 und 180°C in Gegenwart einer Base cyclisiert, verseift und durch Zugabe von Säure die Verbindung der allgemeinen Formel I ausfällt.

5. Verfahren zur Herstellung von Chinoloncarbonsäuren der allgemeinen Formel I 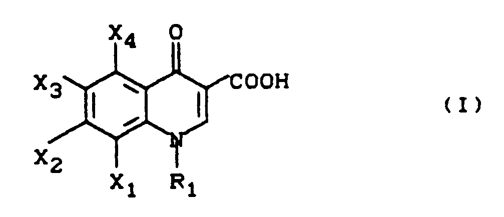 in der
R¹ für Cyclopropyl steht,
X¹ und X² für Chlor steht und
X³ Fluor bedeutet und
X⁴ für Wasserstoff steht,
dadurch gekennzeichnet, daß man ohne Isolierung der Reaktionsprodukte von Zwischenstufen in einer sogenannten Eintopfreaktion eine Verbindung der allgemeinen Formel II 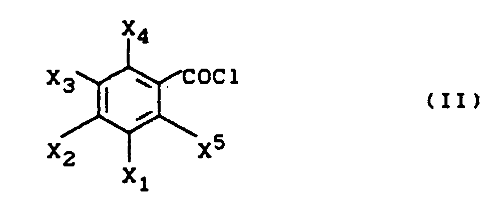 in der X¹ bis X⁴ die obengenannte Bedeutung haben und
X⁵ für Halogen, insbesondere Chlor oder Fluor steht, mit einer Verbindung der allgemeinen Formel III 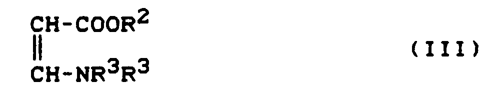 in der
R² und R³ gleich oder verschieden sind und für C₁-C₄-Alkyl stehen,
in Gegenwart eines Lösungsmittels und einer Base, gegebenenfalls unter Erwärmung auf 50 bis 150°C zu einer Verbindung der allgemeinen Formel IV 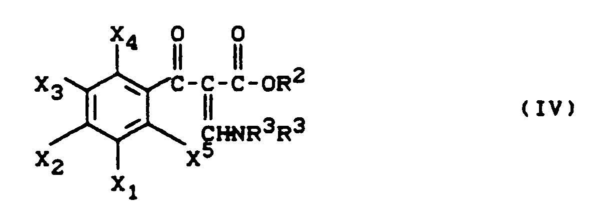 umsetzt,
diese Verbindung IV bei Temperaturen von 50 bis 120°C in Gegenwart der obengenannten Lösungsmittel und in Gegenwart von Cyclopropylamin einem Aminaustausch unterwirft, wobei eine Verbindung der allgemeinen Formel V 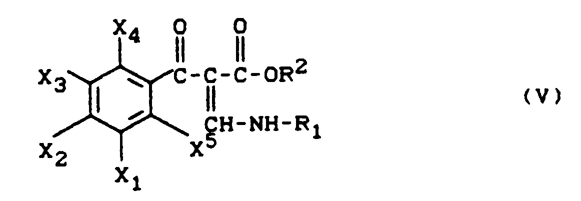 mit den obengenannten Restebedeutungen entsteht, und anschließend die Verbindung V bei Temperaturen zwischen 80 und 180°C in Gegenwart einer Base cyclisiert, verseift und durch Zugabe von Säure die Verbindung der allgemeinen Formel I ausfällt.

## Claims

1. Process for the preparation of quinolonecarboxylic acids of the general formula I 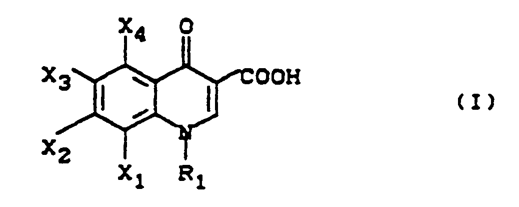 in which
R¹ represents propyl, cyclopropyl, isopropyl or vinyl,
X¹ denotes fluorine, chlorine, Br, CN, NO₂ or hydrogen, and
X², X³ and X⁴ represent fluorine, chlorine, NO₂ or hydrogen,
characterized in that a compound of the general formula II 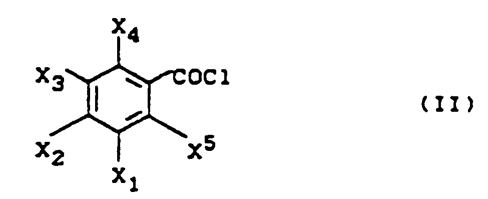 in which X¹ to X⁴ have the abovementioned meaning, and
X⁵ represents halogen, in particular chlorine or fluorine,
is reacted, without isolation of reaction products of the intermediate stages in a so-called one-pot reaction, with a compound of the general formula III 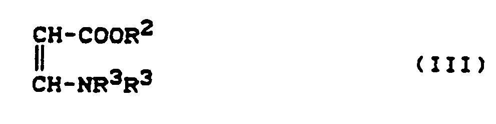 in which
R² and R³ are identical or different and represent C₁-C₄-alkyl,
in the presence of a solvent and of a base, where appropriate heating to 50 to 150°C, to give a compound of the general formula IV 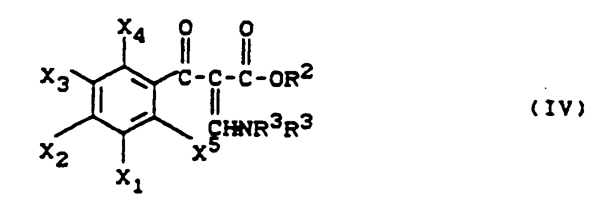 this compound IV is subjected to an amine exchange at temperatures of 50 to 120°C in the presence of the abovementioned solvents and in the presence of an amine of the general formula R₁NH₂ in which R¹ has the abovementioned meaning, resulting in a compound of the general formula V 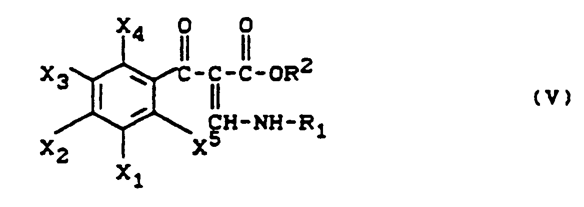 having the abovementioned radical meanings, and subsequently the compound V is cyclized and hydrolysed at temperatures between 80 and 180°C in the presence of a base, and the compound of the general formula I is precipitated by addition of acid.

2. Process for the preparation of quinolonecarboxylic acids of the general formula I 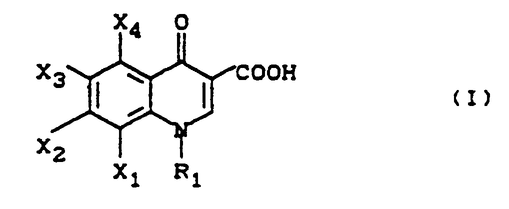 in which
R¹ represents cyclopropyl,
X¹ denotes fluorine, chlorine, Br, CN, NO₂ or hydrogen, and
X², X³ and X⁴ represent fluorine, chlorine, NO₂ or hydrogen,
characterized in that a compound of the general formula II 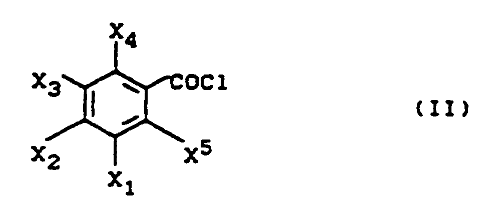 in which X¹ to X⁴ have the abovementioned meaning, and
X⁵ represents halogen, in particular chlorine or fluorine,
is reacted, without isolation of reaction products of the intermediate stages in a so-called one-pot reaction, with a compound of the general formula III 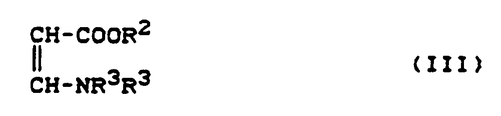 in which
R² and R³ are identical or different and represent C₁-C₄-alkyl,
in the presence of a solvent and of a base, where appropriate heating to 50 to 150°C, to give a compound of the general formula IV 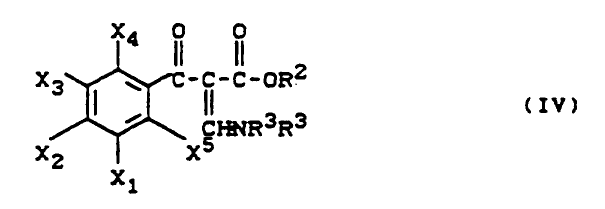 this compound IV is subjected to an amine exchange at temperatures of 50 to 120°C in the presence of the abovementioned solvents and in the presence of cyclopropylamine, resulting in a compound of the general formula V 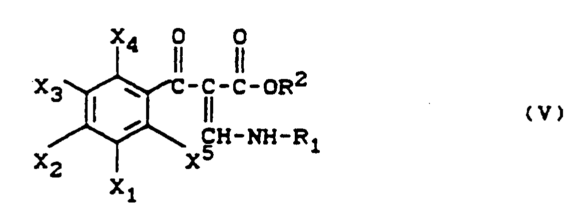 having the abovementioned radical meanings, and subsequently the compound V is cyclized and hydrolysed at temperatures between 80 and 180°C in the presence of a base, and the compound of the general formula I is precipitated by addition of acid.

3. Process for the preparation of quinolonecarboxylic acids of the general formula I 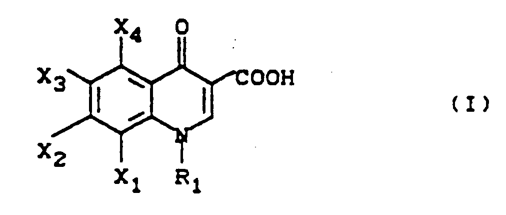 in which
R¹ represents cyclopropyl,
X¹, X² and X³ are identical or different and represent fluorine or chlorine, and
X⁴ represents hydrogen,
characterized in that a compound of the general formula II 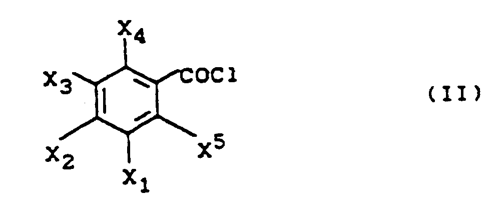 in which X¹ to X⁴ have the abovementioned meaning, and
X⁵ represents halogen, in particular chlorine or fluorine,
is reacted, without isolation of reaction products of the intermediate stages in a so-called one-pot reaction, with a compound of the general formula III 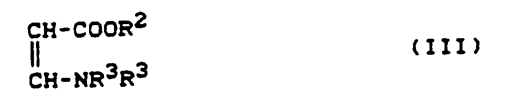 in which
R² and R³ are identical or different and represent C₁-C₄-alkyl,
in the presence of a solvent and of a base, where appropriate heating to 50 to 150°C, to give a compound of the general formula IV 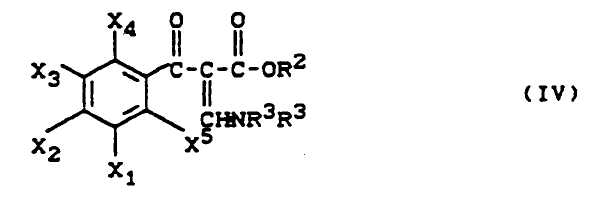 this compound IV is subjected to an amine exchange at temperatures of 50 to 120°C in the presence of the abovementioned solvents and in the presence of cyclopropylamine, resulting in a compound of the general formula V 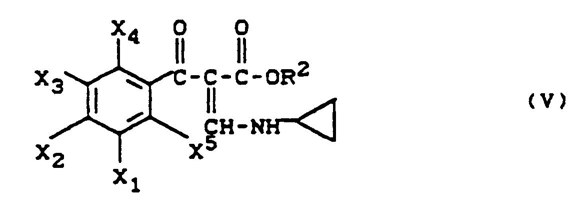 having the abovementioned radical meanings, and subsequently the compound V is cyclized and hydrolysed at temperatures between 80 and 180°C in the presence of a base, and the compound of the general formula I is precipitated by addition of acid.

4. Process for the preparation of quinolonecarboxylic acids of the general formula I 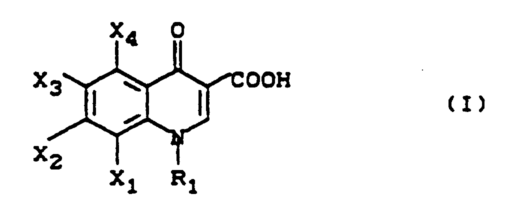 in which
R¹ represents cyclopropyl,
X¹ and X⁴ denote hydrogen,
X₃ represents fluorine, and
X² denotes chlorine,
characterized in that a compound of the general formula II 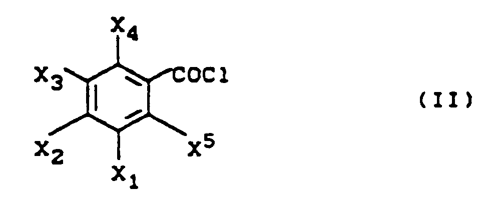 in which X¹ to X⁴ have the abovementioned meaning, and
X⁵ represents halogen, in particular chlorine or fluorine,
is reacted, without isolation of reaction products of the intermediate stages in a so-called one-pot reaction, with a compound of the general formula III 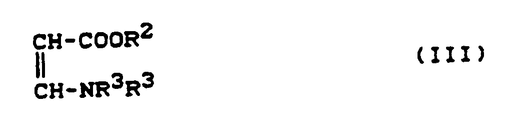 in which
R² and R³ are identical or different and represent C₁-C₄-alkyl,
in the presence of a solvent and of a base, where appropriate heating to 50 to 150°C, to give a compound of the general formula IV 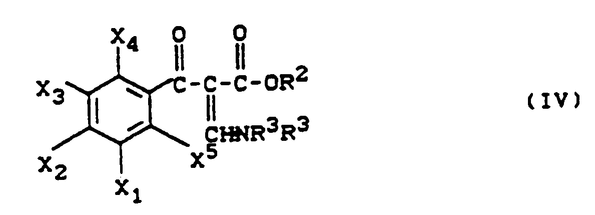 this compound IV is subjected to an amine exchange at temperatures of 50 to 120°C in the presence of the abovementioned solvents and in the presence of cyclopropylamine, resulting in a compound of the general formula V 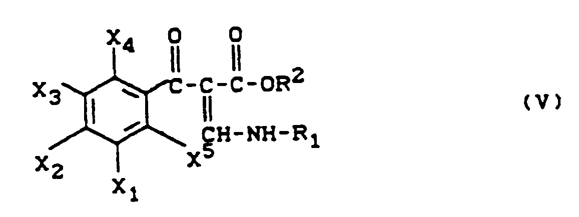 having the abovementioned radical meanings, and subsequently the compound V is cyclized and hydrolysed at temperatures between 80 and 180°C in the presence of a base, and the compound of the general formula I is precipitated by addition of acid.

5. Process for the preparation of quinolonecarboxylic acids of the general formula I 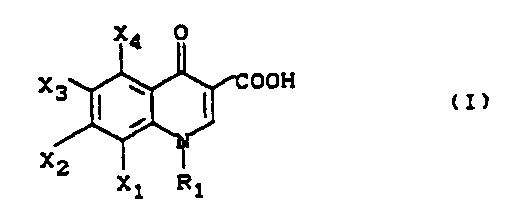 in which
R¹ represents cyclopropyl,
X¹ and X² represent chlorine,
X³ denotes fluorine, and
X⁴ denotes hydrogen,
characterized in that a compound of the general formula II 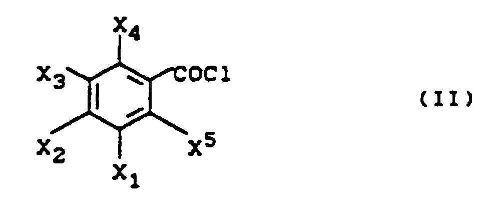 in which X¹ to X⁴ have the abovementioned meaning, and
X⁵ represents halogen, in particular chlorine or fluorine,
is reacted, without isolation of the reaction products of intermediate stages in a so-called one-pot reaction, with a compound of the general formula III 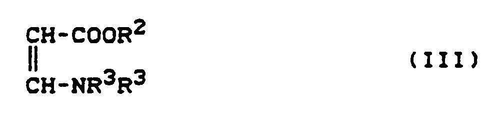 in which
R² and R³ are identical or different and represent C₁-C₄-alkyl,
in the presence of a solvent and of a base, where appropriate heating to 50 to 150°C, to give a compound of the general formula IV 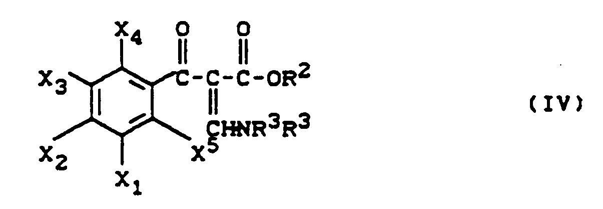 this compound IV is subjected to an amine exchange at temperatures of 50 to 120°C in the presence of the abovementioned solvents and in the presence of cyclopropylamine, resulting in a compound of the general formula V 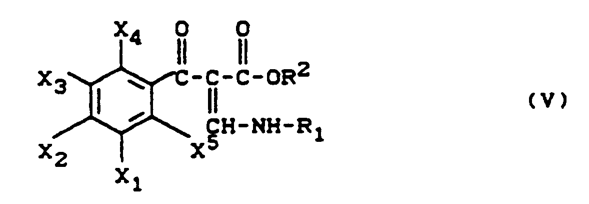 having the abovementioned radical meanings, and subsequently the compound V is cyclized and hydrolysed at temperatures between 80 and 180°C in the presence of a base, and the compound of the general formula I is precipitated by addition of acid.

## Revendications

1. Procédé de production d'acides quinolonecarboxyliques de formule générale I 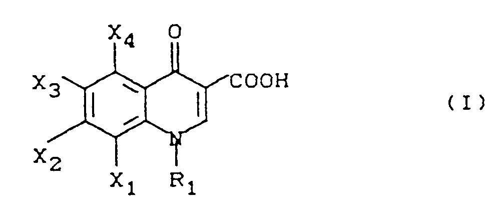 dans laquelle
R¹ est un groupe propyle, cyclopropyle, isopropyle ou vinyle,
X¹ est du fluor, du chlore, du brome, un groupe CN, NO₂ ou de l'hydrogène et
X², X³ et X⁴ représentent du fluor, du chlore, un groupe NO₂ ou de l'hydrogène,
caractérisé en ce qu'on fait réagir sans isolement des produits réactionnels des stades intermédiaires, dans une réaction dite en récipient unique, un composé de formule générale II 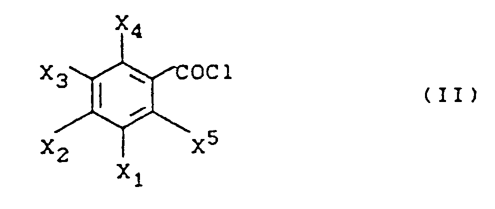 dans laquelle X¹ à X⁴ ont la définition indiquée ci-dessus et
X⁵ représente un halogène, en particulier le chlore ou le fluor, avec un composé de formule générale III 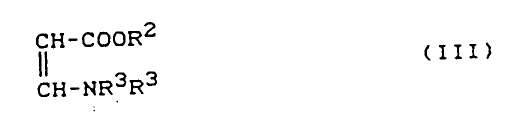
dans laquelle
R² et R³ sont égaux ou différents et représentent un groupe alkyle en C₁ à C₄,
en présence d'un solvant et d'une base, éventuellement avec chauffage à une température de 50 à 150°C pour former un composé de formule générale IV 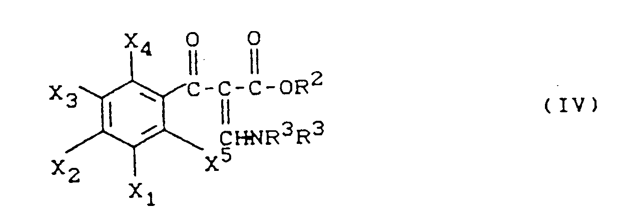 on soumet ce composé IV, à des températures de 50 à 120°C en présence des solvants indiqués ci-dessus et en présence d'une amine de formule générale R₁NH₂, dans laquelle R¹ a la définition indiquée ci-dessus, à un échange d'amine, et il se forme alors un composé de formule générale V 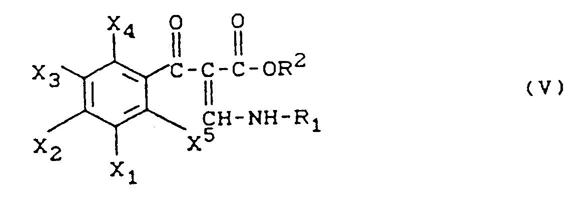 dans laquelle les restes ont les définitions indiquées ci-dessus, puis on cyclise le composé V à des températures comprises entre 80 et 180°C en présence d'une base, on le saponifie et on précipite le composé de formule générale I par addition d'acide.

2. Procédé de production d'acides quinolonecarboxyliques de formule générale I 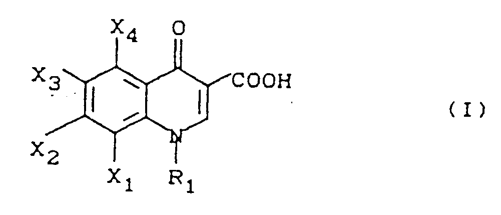 dans laquelle
R¹ est un groupe cyclopropyle,
X¹ représente le fluor, le chlore, le brome, un groupe CN, NO₂ ou l'hydrogène,
X², X³ et X⁴ représentent du fluor, du chlore, un groupe NO₂ ou de l'hydrogène,
caractérisé en ce qu'on fait réagir sans isolement des produits réactionnels des stades intermédiaires, dans une réaction dite en récipient unique, un composé de formule générale II 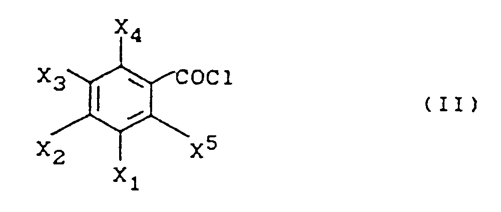 dans laquelle X¹ à X⁴ ont la définition mentionnée ci-dessus et
X⁵ représente un halogène, en particulier le chlore ou le fluor, avec un composé de formule générale III 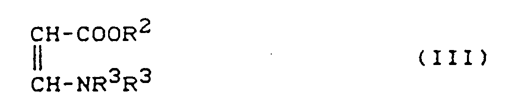
dans laquelle
R² et R³ sont égaux ou différents et représentent un groupe alkyle en C₁ à C₄,
en présence d'un solvant et d'une base, le cas échéant en chauffant à une température de 50 à 150°C, pour former un composé de formule générale IV 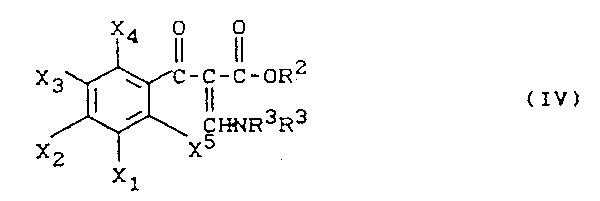 on soumet ce composé IV, à des températures de 50 à 120°C en présence des solvants mentionnés ci-dessus et en présence de cyclopropylamine, à un échange d'amine, et il se forme ainsi un composé de formule générale V 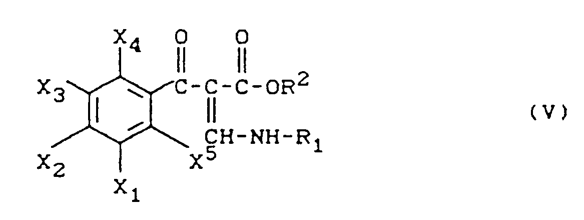 dans laquelle les restes ont les définitions mentionnées ci-dessus, puis on cyclise le composé V à des températures comprises entre 80 et 180°C en présence d'une base, on le saponifie et on précipite le composé de formule générale I par addition d'acide.

3. Procédé de production d'acides quinolonecarboxyliques de formule générale I 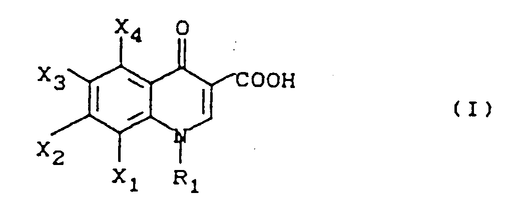 dans laquelle
R¹ est un groupe cyclopropyle,
X¹, X² et X³ sont égaux ou différents et représentent du fluor ou du chlore et
X⁴ est de l'hydrogène,
caractérisé en ce qu'on fait réagir, sans isolement des produits réactionnels des stades intermédiaires, dans une réaction dite en récipient unique, un composé de formule générale II 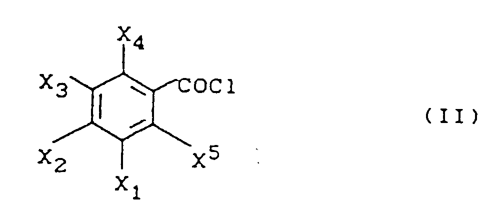 dans laquelle X¹ à X⁴ ont la définition indiquée ci-dessus et
X⁵ représente un halogène, en particulier le chlore ou le fluor, avec un composé de formule générale III 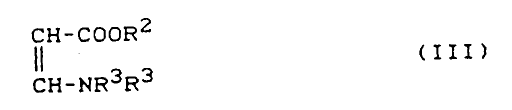
dans laquelle
R² et R³ sont égaux ou différents et représentent un groupe alkyle en C₁ à C₄,
en présence d'un solvant et d'une base, éventuellement en chauffant à une température de 50 à 150°C pour former un composé de formule générale IV 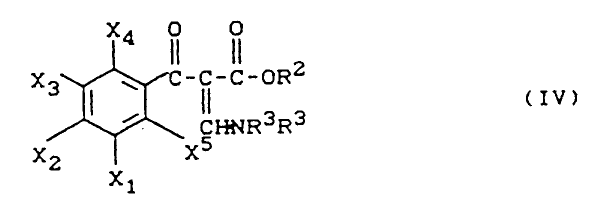 on soumet ce composé IV, à des températures de 50 à 120°C en présence des solvants mentionnés ci-dessus et en présence de cyclopropylamine, à un échange d'amine, et on obtient ainsi un composé de formule générale V 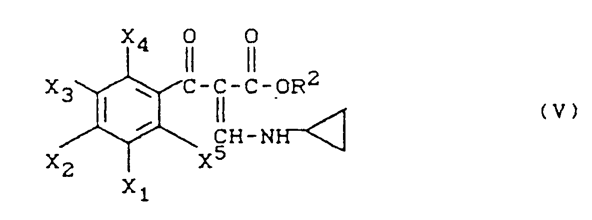 dans laquelle les restes ont les définitions mentionnées ci-dessus, puis on cyclise le composé V à des températures comprises entre 80 et 180°C en présence d'une base, on le saponifie et on précipite le composé de formule générale I par addition d'acide.

4. Procédé de production d'acides quinolonecarboxyliques de formule générale I 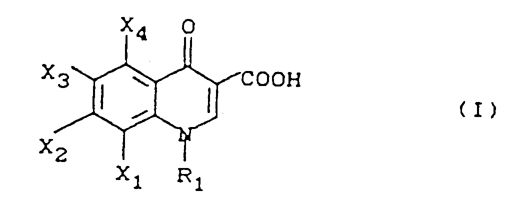 dans laquelle
R¹ est un groupe cyclopropyle,
X¹ et X² représentent de l'hydrogène,
X₃ est du fluor et
X² est du chlore,
caractérisé en ce qu'on fait réagir, sans isolement des produits réactionnels des stades intermédiaires, dans une réaction dite en récipient unique, un composé de formule générale II 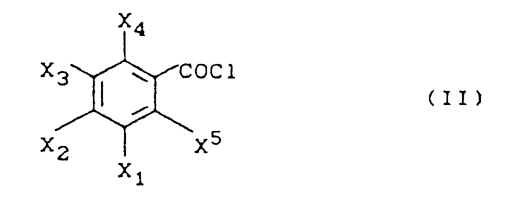 dans laquelle X¹ à X⁴ ont la définition indiquée ci-dessus et
X⁵ représente un halogène, en particulier le chlore ou le fluor, avec un composé de formule générale (III) 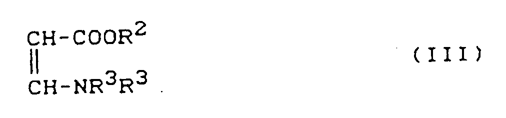
dans laquelle
R² et R³ sont égaux ou différents et représentent un groupe alkyle en C₁ à C₄,
en présence d'un solvant et d'une base, éventuellement en chauffant à une température de 50 à 150°C pour former un composé de formule générale IV 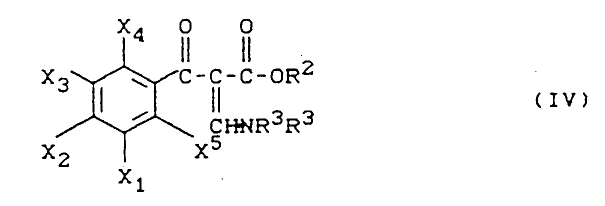 on soumet ce composé IV, à des températures de 50 à 120°C en présence des solvants mentionnés ci-dessus et en présence de cyclopropylamine, à un échange d'amine, et il est ainsi formé un composé de formule générale V 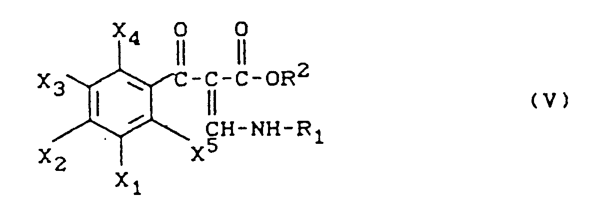 dans laquelle les restes ont les définitions mentionnées ci-dessus, puis on cyclise le composé V à des températures comprises entre 80 et 180°C en présence d'une base, on le saponifie et on précipite le composé de formule générale I par addition d'acide.

5. Procédé de production d'acides quinolonecarboxyliques de formule générale I 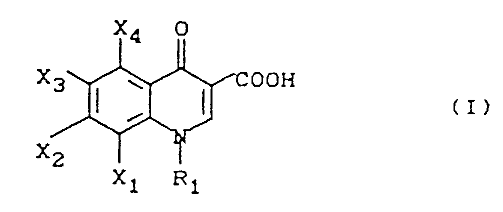 dans laquelle
R¹ est un groupe cyclopropyle,
X¹ et X² représentent du chlore,
X³ est du fluor et
X⁴ est de l'hydrogène,
caractérisé en ce qu'on fait réagir, sans isolement des produits réactionnels des stades intermédiaires, dans une réaction dite en récipient unique, un composé de formule générale II 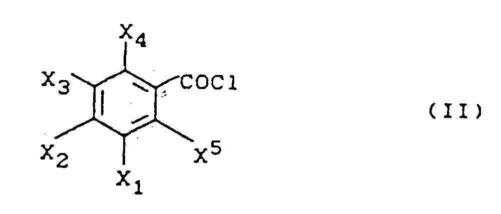 dans laquelle X¹ à X⁴ ont la définition indiquée ci-dessus et
X⁵ représente un halogène, en particulier le chlore ou le fluor, avec un composé de formule générale (III) 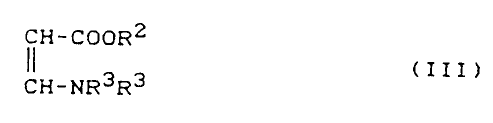
dans laquelle
R² et R³ sont égaux ou différents et représentent un groupe alkyle en C₁ à C₄,
en présence d'un solvant et d'une base, éventuellement en chauffant à une température de 50 à 150°C pour former un composé de formule générale IV 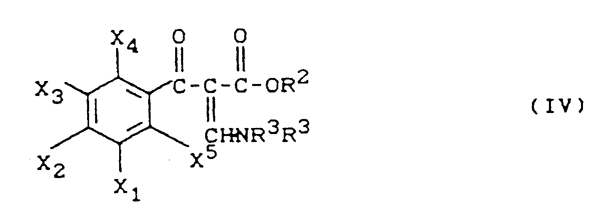 on soumet ce composé IV, à des températures de 50 à 120°C en présence des solvants mentionnés ci-dessus et en présence de cyclopropylamine, à un échange d'amine, et il se forme alors un composé de formule générale V 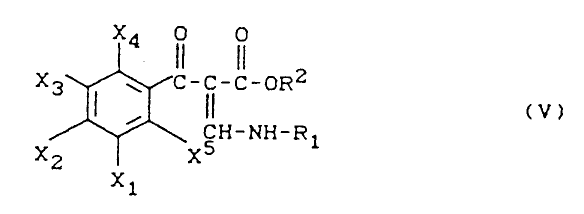 dans laquelle les restes ont les définitions mentionnées ci-dessus, puis on cyclise le composé V à des températures de 80 à 180°C en présence d'une base, on le saponifie et on précipite le composé de formule générale I par addition d'acide.
